**Europäisches Patentamt**

**European Patent Office**

**Office européen des brevets**

(19)

(11) Veröffentlichungsnummer: **0 111 631**
**B1**

(12) **EUROPÄISCHE PATENTSCHRIFT**

(45) Veröffentlichungstag der Patentschrift:
23.11.88

(21) Anmeldenummer: 83108709.3

(22) Anmeldetag: 05.09.83

(51) Int. Cl.⁴: **A 61 K 7/06,** A 61 K 7/48

(54) Biologisches Mittel zur Behandlung der Haare und der Haut.

(30) Priorität: 16.11.82 DE 3242446

(43) Veröffentlichungstag der Anmeldung:
27.06.84 Patentblatt 84/26

(45) Bekanntmachung des Hinweises auf die Patenterteilung:
23.11.88 Patentblatt 88/47

(84) Benannte Vertragsstaaten:
AT BE CH DE FR GB IT LI LU NL SE

(56) Entgegenhaltungen:
FR-A-1 288 708
FR-A-1 503 586
US-A-2 320 478

(73) Patentinhaber: Sidhu, T.S., Adam- Vogt- Strasse 8,
D-8910 Landsberg/Lech (DE)

(72) Erfinder: Sidhu, T.S., Adam- Vogt- Strasse 8,
D-8910 Landsberg/Lech (DE)

(74) Vertreter: Kohler, Anton, Dr., Dr.A.Kohler +
M.Schroeder Patentanwälte Schellingstrasse 33,
D-8000 München 40 (DE)

EP 0 111 631 B1

## Beschreibung

Die Erfindung betrifft ein biologisches Mittel zur Behandlung der Haare und der Haut.

Das Wachstum der Haare und die Alterung der Haut stehen in einem ursächlichen Zusammenhang mit dem Hormonhaushalt des Körpers. Haarausfall betrifft vor allem Männer, jedoch auch bisweilen Frauen nach großen Veränderungen im Hormonhaushalt, wie z.B. nach Schwangerschaften oder bestimmten medikamentösen Behandlungen.

Es sind zahlreiche Präparate und Literaturstellen für Mittel zur Behandlung oder Regeneration der Haare und der Kopfhaut bekannt.

Derartige haarwuchsfördernde oder hautstraffende Mittel sind meistens auf der Basis von Alkoholen oder Ölen aufgebaut und lassen sich grob aufgrund der darin enthaltenen Wirkstoffe in drei Gruppen unterteilen:

1) Hormone, insbesondere Steroide, z. B. Testosteron gemäß der DE-PS-1 617 857, Kombinationen von Östrogenen und Gestagenen gemäß der europäischen Patentanmeldung 33 164, humanes Placenta-Lactogen gemäß der DE-OS-2 540 971.

2) Vitamine wie z. B. Biotin (Vitamin H), Pantothensäure (Vitamin $B_5$) und änliche Verbindungen wie Cholin und Betain gemäß der DE-AS-1 007 957.

3) Mittel, welche der Durchblutung der Kopfhaut dienen, wie z.B. Roßkastanienextrakt gemäß der DE-OS-1 492 193 Meerrettichextrakt und Extrakt von Senfkörnern gemäß der DE-OS-3 039 281, alkoholische Extrakte von Paprika gemäß der DE-OS-2 649 846 und ähnliche Materialien.

Weitere bekannte Mittel stellen Brennesselextrakt, Birkensaft, Cholesterin und dergleichen dar, ohne daß die vorstehende Aufzählung bei der großen Anzahl von Vorschlägen vollständig sein kann.

Extrakte aus Beinwell (Symphytum officinale) werden als Antiphlogistica und in Gesichtswässern angewandt. Allantoin wird in niedrigen Konzentrationen in kosmetischen Präparaten eingesetzt.

Nach dem gegenwärtigen Stand des Wissens (K. Schrader, Grundlagen und Rezepturen der Cosmetica, 1979) werden bei Haarproblemen zwar mit hormonhaltigen Mitteln gewisse Erfolge erzielt, jedoch treten dabei auf die Dauer Nebenwirkungen auf.

Gemäß der französischen Patentschrift 1 288 708 wird aus dem Riesenbovist eine alkoholunlösliche "wirksame Substanz" isoliert, über deren Wirksamkeitsrichtung nichts zu entnehmen ist. In der französischen PS 1 503 586 werden Fettkörperextrakten bemerkenswerte Beschleunigungseigenschaften für die Zellproduktion zugeschrieben, die sie für kosmetische Produkte zur Regeneration geeignet machen sollen.

Selbst wenn man lediglich die deutsche Patentliteratur durchsieht, scheinen alle Haarprobleme zur vollen Zufriedenheit gelöst zu sein. Genau die gegenteilige Ansicht wird jedoch in der Fachliteratur und von den Betroffenen geäußert. Übereinstimmend sind beide der Ansicht, daß es ein zuverlässiges Mittel zur Regeneration des Haarwuchses und zur Regeneration der Haut bisher nicht gibt. Die besten bisher noch verwendeten Mittel enthalten Steroide, bei welchen auf die Dauer schwerwiegende Nebenwirkungen auftreten.

Die Aufgabe der vorliegenden Erfindung besteht somit in einem biologischen Mittel, welches eine Aktivierung der Haarwurzeln und der Haut herbeiführt und dadurch die grundlegenden Voraussetzungen für Kopfhautregeneration und/oder Haarwuchs bewirkt sowie ein Verfahren zu dessen Herstellung.

Gegenstand der Erfindung ist somit ein biologisches Mittel zur Behandlung der Kopfhaut und der Haare, gekennzeichnet durch einen Extrakt auf der Basis hautverträglicher Alkohole oder Öle von

1) Fruchtkörpern von ungiftigen Ständerpilzen (Basidiomyceten), ausgenommen Calvatia maxima, und

2) Insektenlarven, die sich von Pilzen ernähren, sowie deren Stoffwechselprodukten und gegebenenfalls

3) weiteren Zusätzen.

Ein weiterer Gegenstand der Erfindung ist ein Verfahren zur Herstellung dieses biologischen Mittels, das dadurch gekennzeichnet ist, daß ein Gemisch aus zerkleinerten Fruchtktkörpern von ungiftigen Ständerpilzen (Basidiomyceten), ausgenommen Calvatia maxima, und Insektenlarven, die sich von Pilzen ernähren, während eines Zeitraums von einigen Stunden bis zu einigen Tagen stehengelassen wird und anschließend mit einem hautverträglichen alkoholischen oder öligen Extraktionsmittel kontaktiert wird und der erhaltene Extrakt abgetrennt wird und gegebenenfalls mit weiteren Zusätzen aus der Gruppe von alkoholischen Extrakten aus Moosen, alkoholischen Extrakten aus Beinwell (Symphytum officinale) und Allantoin als wirksame Komponenten versetzt ist.

Man erhält somit Mittel, die bei ihrer Anwendung auf die Kopfhaut oder die gealterte Haut bereits nach drei Tagen ihre Wirkung erkennen lassen und positiv gegen (A) Haarausfall, Schuppenbildung und vermehrten Fettfluß der Kopfhaut wirken. In speziellen Ausführungsformen ergibt sich eine (B) Regenerierung der Kopfhaut, so daß der Haarwuchs wieder eintritt. Die regenerierende Wirkung des Mittels läßt sich insbesondere daraus ersehen, daß der Haarwuchs praktisch in seiner ursprünglichen Farbe wieder einsetzt.

Als ein Ausgangsmaterial für den herzustellenden Extrakt dienen die Fruchtkörper von ungiftigen Ständerpilzen (Basidiomyceten), ausgenommen Calvatia maxima. Es können praktisch sämtliche Arten und Klassen von ungiftigen Ständerpilzen verwendet werden, jedoch werden günstigerweise die Pilzfruchtkörper der Familien Agaricales und/oder Boletales verwendet. Besonders günstig erwiesen sich die Arten Egerling (Agaricus), Täubling (Russula), Filzröhrlinge (Xerocomus) und Hallimasch (Armillariella). Aufgrund ihrer Zugänglichkeit und hervorragenden Eignung werden die Arten Maronenröhrling (Xerocomus badius) oder Hallimasch (Armillariella mellea) besonders bevorzugt.

Für die speziellen Ausführungsformen der Regenerierung besteht der zweite Ausgangsstoff für das Mittel gemäß der Erfindung aus Insektenlarven oder -maden, die sich von Pilzen ernähren. Bevorzugt bestehen diese Insektenlarven aus Fliegenlarven, wobei die Fliegenlarven der Familien Lycoriidae (Sciaridae, Trauermücken) und/ oder Fungivoridae (Mycetophilidae, Pilzmücken) besonders bevorzugt werden.

Ein weiterer bevorzugter Bestandteil des Mittels sind alkoholische Extrakte aus Moosen, die an Bäumen an Holz wachsen. Obwohl sich eine große Vielzahl derartiger Moose eignet, werden folgende Moosarten bevorzugt: Schönes Widertonmoos (Polytrichum formosum), Gabelzahnmoos (Dicranum scoparium), Silber-Birnmoos (Bryum argenteum) und Schwarzes Eichhornmoos (Lencodon scinroides). Dieser Extrakt zeigt bereits als solcher neben der Wirkung gegen Haarausfall und Schuppenbildung eine gewisse regenerierende Wirkung zur Ausbildung von neuem Haarwuchs.

Als weiterer Zusatz erwies sich ein alkoholischer Extrakt des Beinwell (Symphytum officinale) als günstig. Weiterhin ergab der Zusatz von Allantoin entweder als Substanz oder als alkoholische Lösung in einer Menge als Feststoff von 0,2 bis 3 Gew.-% des Mittels günstige Wirkungen, wobei Mengen von etwa 1 bis 2 Gew.-% bevorzugt werden. Allantoin kann als solches oder in Salzform, beispielsweise als Aluminiumsalz oder -komplex, vorliegen. Die Konzentrationen der alkoholischen Extrakte werden, gegebenenfalls durch Wasserzusatz, so eingeregelt, daß sich eine Alkoholkonzentration des fertigen Mittels von 30 bis 50 Volumen-%, vorzugsweise in der Gegend von etwa 40 Volumen-%, ergibt.

Durch die vorstehend aufgeführten Extrakte und ihre Kombinationen ergeben sich sehr wirksame Mittel zur Behandlung der Haare und der Kopfhaut. Haarausfall, übermäßige Fettabscheidung und Schuppenbildung werden durch alkoholischen Pilzextrakt, Moosextrakt, Pilzextrakt-Moosextrakt-Kombination, Pilzextrakt-Beinwellextrakt-Kombination, Pilzextrakt-Allantoin-Kombination sowie Pilz-Larven-Ölextrakt verhindert. Ein völlig überraschender . Effekt wird bei Mitverwendung von alkoholischem Pilz-Larvenextrakt erhalten, indem offensichtlich synergistisch eine völlige Regeneration der Kopfhaut auftritt und erneuter Haarwuchs einsetzt. Diese bevorzugten Mittel bestehen aus jeweils alkoholischem Pilz-Larven-Extrakt, Pilz-Larven-Moos-Extrakt, Pilz-Larven-Beinwell-Extrakt, Pilz-Larven-Allantoin-Extrakt, Pilz-Larven-Moos-Beinwell-Extrakt und Pilz-Larven-Moos-Beinwell-Allantoin-Extrakt.

Das Verfahren zur Herstellung des biologischen Mittels besteht prinzipiell darin, daß ein Gemisch aus zerkleinerten Fruchtkörpern von ungiftigen Ständerpilzen (Basidiomyceten), ausgenommen Calvatia maxima, und Insektenlarven, die sich von Pilzen ernähren, während eines Zeitraums von einigen Stunden bis zu einigen Tagen stehengelassen wird und anschließend mit einem hautverträglichen Extraktionsmittel kontaktiert wird und der erhaltene Extrakt abgetrennt wird und gegebenenfalls mit den weiteren Zusätzen aus der Gruppe von alkoholischen Extrakten aus Moosen, alkoholischen Extrakten aus Beinwell (Symphytum officinale) und Allantoin versetzt wird.

Das Extraktionsmittel muß hautverträglich sein. Als alkoholische Extraktionsmittel werden Äthanol und Isopropanol bevorzugt.

Im einzelnen wird der Pilzextrakt aus zerkleinerten Fruchtkörpern der oben angegebenen Pilzarten durch Extraktion mit Äthanol und/oder Isopropanol, gegebenenfalls unter Wasserzusatz, in an sich bekannter Weise bei 20 bis 40° C während einiger Tage bis Wochen hergestellt. Für Einzelheiten wird auf die weiter unten abgehandelte Herstellungsweise des Pilz-Larven-Extraktes verwiesen.

Der Moosextrakt wird aus Moosen bevorzugt vom Typ Bryophyta, insbesondere der Art Funaria, gewonnen, wobei die vier vorstehend aufgeführten Moose Poytrichum formosum, Dicranum scoparium, Bryum argenteum und Lencodon scinroides bevorzugt werden. Die frisch gewonnenen Moose werden einzeln oder als Gemische mittels 30 bis 60 %-igem Alkohol etwa bei Raumtemperatur während eines Zeitraums bis zu 2 Stunden unter Schütteln behandelt, dann abgepreßt und auf die gewünschte Alkoholkonzentration gebracht.

Die gleiche Arbeitsweise wie bei der Gewinnung des Moosextraktes gilt auch prinzipiell für die Gewinnung des alkoholischen Extraktes aus Beinwell, der in Form der Wurzeln, der Stengel oder der ganzen Pflanzen zur Anwendung gelangen kann.

Im folgenden wird die bevorzugte Ausführungsform der Herstellung des Pilz-Larvenextraktes beschrieben.

Das Verfahren zur Herstellung geht von gut entwickelten Fruchtkörpern der vorstehend aufgeführten Pilzarten aus, die mit den Larven, insbesondere der Trauer- und/ oder Pilzmücken, versetzt sind. In aller Regel weisen die Fruchtkörper bereits einen Befall durch derartige Larven auf und derartige Fruchtkörper werden bevorzugt eingesetzt. Man kann auch beispielsweise auf Hallimasch stark entwickelte Kulturen dieser Larven oder Maden auf weniger befallene Fruchtkörper übertragen oder man kann in geeigneten Medien wie Laub entwickelte Kolonien der Fliegenlarven auf die günstigerweise fein zerkleinerten Fruchtkörper übertragen.

Das erhaltene Gemisch wird im zerkleinerten Zustand in einem für Luftzutritt offenen Behälter stehengelassen, wobei sich die Entwicklungszeit je nach dem Entwicklungszustand der Larven zwischen einigen Stunden und etwa 60 Stunden, vorzugsweise 10 bis 48 Stunden, regelt. Die Temperatur liegt dabei etwa bei Raumtemperatur in der Gegend von etwa 15 bis 25°C. Es können jedoch auch etwas erhöhte Temperaturen angewandt werden. Während dieser Zeit entwickeln sich die in dem Pilzmaterial enthaltenen Larven äußerst stark und die sich immer kräftiger entwickelnden und vermehrenden Larven haben einen großen Teil der Pilzsubstanz verzehrt und wieder ausgeschieden. Der Ansatz hat sich zu diesem Zeitpunkt dunkelbraun bis schwarz verfärbt.

Wenn die Entwicklung der Larven den Höhepunkt erreicht hat, gibt man zur Herstellung eines alkoholischen Extraktes einen hochprozentigen wäßrigen Alkohol wie Äthanol oder Isopropanol hinzu, dessen Konzentration 60 bis 80 % beträgt, wobei eine etwa 70 %-ige Konzentration des wäßrigen Alkohols bevorzugt wird. Hierdurch werden die Larven sofort abgetötet.

Die bei der Extraktion anzuwendende Konzentration der Alkohole hängt in erster Linie von dem Zustand des zu extrahierenden Substrates und der in dem Mittel schon aus Konservierungsgründen zu erzielenden Konzentration des Alkohols von rund 30 bis 50 %, insbesondere 40 %, ab. Zudem ist auch das umgesetzte Pilzmaterial eine ziemlich flüssige Masse, so daß auch die handelsüblichen, relativ hohen Konzentrationen angewandt werden können, so daß sich ein Mittel mit einer zur Konservierung ausreichenden Alkoholkonzentration ergibt.

Das Gemisch wird gründlich durchmischt und der Behälter verschlossen. Man läßt den Behälter während längerer Zeit gegebenenfalls unter wiederholtem Durchmischen bei einer Temperatur von etwa 20 bis 40°C ruhen. Im allgemeinen ist ein Zeitraum von einigen Tagen bis zu 50 Tagen ausreichend, wobei ein Zeitraum von 25 bis 40 Tagen sich als besonders günstig erwies. Bei intensiveren Extraktionsverfahren läßt sich dieser Zeitraum erheblich verkürzen. Anschließend wird der wäßrig-alkoholische Extrakt von den verbliebenen Feststoffen abgetrennt, was durch übliche Maßnahmen wie Abpressen, Abzentrifugieren oder Abfiltrieren erfolgen kann. Man kann die Extraktion auch in einem der üblichen Extraktoren durchführen.

Zur Herstellung eines Extraktes aus Pilzen und Larven auf der Basis von Ölen können sämtliche hautverträglichen Öle verwendet werden, d.h.

pflanzliche, tierische und mineralische Öle. Besonders bevorzugte Beispiele derartiger Öle sind Olivenöl, Sesamöl, Sonnenblumenöl, Sojaöl, Palmöl oder Weißöl (Vaselinöl), sowie Gemische davon. Das Öl wird zu dem Fruchtkörper-Larven-Gemisch zugesetzt und gründlich vermischt und unmittelbar auf etwa 100°C erhitzt, worauf es einige Stunden bei 70 bis 100°C stehengelassen wird. Anschließend wird es einige Tage in einem geschlossenen Behälter stehengelassen, worauf dann der Ölextrakt durch übliche Maßnahmen von dem Feststoff abgetrennt wird, wie z. B. durch Abpressen, Absieben oder Abzentrifugieren oder Preßfiltrieren.

Im allgemeinen wendet man in beiden Fällen etwa gleiche Volumina Pilz-Larven-Material und Extraktionsmittel an.

Der auf die vorstehend beschriebene Weise erhaltene alkoholische Pilz-Larven-Extrakt stellt eine dunkelbraune Flüssigkeit dar und wird bevorzugt zur Regeneration des Haarbodens verwendet.

Der erhaltene Ölextrakt ist eine gelbe ölige Flüssigkeit, die bevorzugt zur Hautbehandlung und Hautstraffung eingesetzt wird und Wirksamkeit gegen Haarausfall, Schuppenbildung und vermehrten Fettfluß zeigt. Er ergibt auch eine Straffung der Haut und in vielen Fällen eine Beseitigung von Hautflecken.

Die alkoholischen Pilz-Larven-Extrakte werden durch Zusatz der vorstehend abgehandelten alkoholischen Moosextrakte und/oder alkoholischen Beinwellextrakte und/oder Allantoin noch weiter in ihrer Wirksamkeit gesteigert.

Die jeweiligen Volumenverhältnisse der Komponenten richten sich nach der gewählten Kombination und dem zu behandelnden Haar- oder Kopfhauteffekt.

Obwohl bereits ein ausgezeichneter Effekt im Hinblick auf Haarausfall, Schuppenbildung, Fettfluß der Kopfhaut und Neuwuchs der Haare mit dem alkoholischen Pilz-Larven-Extrakt (30 bis 50, insbesondere etwa 40 Volumen-% Alkohol) erzielt wird, wird ein praktisch gleich großer Effekt bei Anwendung einer Kombination mit etwa 40 % Volumenteilen Alkohol aus Pilz-Larven-Extrakt und Moosextrakt im Volumenverhältnis von 1 : 1 erzielt.

Eine Verbesserung des Effektes konnte noch bei der Kombination Pilz-Larven-Extrakt (30 bis 45, bevorzugt 40 Volumenteile), Moosextrakt (45 bis 35 Volumenteile) und Beinwellextrakt (15 bis 20 Volumenteile) erzielt werden. In diesen Mitteln können jeweils noch 0,2 bis 3 Gew.-% Allantoin enthalten sein.

Selbstverständlich können sowohl den alkoholischen als auch den ölartigen Mitteln noch weitere Additive wie Konservierungsmittel und insbesondere Aromastoffe gegebenenfalls zugesetzt werden.

Die alkoholischen Mittel auf der Basis Pilz-Larven-Extrakt beenden den Haarausfall und sind selbst bei Haarausfall infolge medikamentöser Nebenwirkungen wirksam. Die Haare werden weich, glänzend und kräftig und gewinnen sogar die ursprüngliche Haarfarbe wieder zurück. Selbst bei lang bestehender Glatze sorgen sie für erneuten Haarwuchs und wirken gegen verstärkte Kopfhautfettung und Talgabsonderung (Seborrhoea). Es hat den Anschein, daß eine totale Regeneration des Haares und des Haarbodens bewirkt wird.

Die Wirkungsweise des Mittels ist noch nicht geklärt. Es wird angenommen, daß es den Haarboden auflockert und die Poren in der Tiefe reinigt. Dadurch werden Schlackenstoffe entfernt, so daß die Haarwurzeln nicht mehr zusammengepreßt sind, sondern frei liegen und die Wirkstoffe des Mittels aufnehmen können. Dadurch wird der Stoffwechsel des Haarbodens und der Haarwurzeln reaktiviert, so daß die Nähr- und Wuchsstoffe wirksam werden können.

Die folgenden Beispiele dienen zur weiteren Erläuterung der Erfindung, ohne auf diese Beispiele beschränkt zu sein.

**Beispiel 1**

2 kg ausgewachsene Exemplare des Maronenröhrlings wurden von anhaftenden Verunreinigungen befreit und dann der Hut mit Röhrenfutter, der Stiel und die Stielbasis grob zerkleinert. Zumindest ein Teil der Pilzkörper zeigte Madenbefall von Pilzmücken und/oder Trauermücken. Das zerkleinerte Material wurde in einen Behälter gegeben, der 24 Stunden offen in einem dunklen Raum bei einer Temperatur von 15 bis 20°C stehengelassen wurde. Es entwickelten sich die dem Röhrling eigenen Pilzmaden, die einen grauen Körper und einen dunklen Kopf hatten. Nach weiteren 24 Stunden hatten die immer kräftiger sich entwickelnden und vermehrenden Maden einen großen Teil des Pilzmaterials verzehrt und ausgeschieden. Das erhaltene Material war dunkelbraun bis schwarz gefärbt.

Zu dem erhaltenen Produkt wurden bei Raumtemperatur sehr schnell 1,5 l reiner, wäßriger, 70 %-iger Alkohol zugegeben, wodurch die Larven sofort abgetötet wurden. Das Material wurde gut durchmischt und der Behälter verschlossen. Er wurde in einem temperierten Raum bei einer Temperatur von 20 bis 4°C 40 Tage stehengelassen.

Anschließend wurde das Gemisch in eine Presse gegeben und abgepreßt und der Extrakt gewonnen. Der Extrakt hatte einen Alkoholgehalt von etwa 50 % und stellte eine dunkelbraune Flüssigkeit dar.

Zur Anwendung als Regerationsmittel für den Haarwuchs werden täglich etwa 10 bis 50 Tropfen verwendet, die in die Kopfhaut und auf die Haare eingerieben werden. Wichtig ist, daß zweimal täglich, nach der Einreibung oder auch später, die Kopfhaut mit gewöhnlichem Wasser leicht befeuchtet wird. Die Wirkung beginnt sich nach etwa 3 Tagen zu zeigen und spätestens nach einigen Wochen beginnt der Haarnachwuchs. Im allgemeinen setzt man die Behandlung 6 Wochen lang fort. Es zeigt sich ein kräftiger Haarnachwuchs, der praktisch in der ursprünglichen Farbe auftritt. Nebenwirkungen wurden keine beobachtet. Diese Wirkung wird als Wirkung B bezeichnet, nämlich Regenerierung des Haarwuchses. Sie tritt zusätzlich zur Wirksamkeit gegen Haarausfall, Schuppenbildung und Fettfluß der Kopfhaut auf, wobei die letztere Wirksamkeit als Wirksamkeit A bezeichnet wird.

**Beispiel 2**

Es wurde entsprechend Beispiel 1 gearbeitet, jedoch 2 kg Fruchtkörper von Hallimasch als Ausgangsmaterial eingesetzt. Die sich auf diesem Material entwickelnden Maden hatten einen weißen Körper und einen schwarzen Kopf. Die Gewinnung des Extraktes erfolgte wie in Beispiel 1.

Es wurde ein Produkt erhalten, das die gleichen Eigenschaften wie das Produkt von Beispiel 1 hatte.

**Beispiel 3**

2 kg Maronenröhrlinge wurden wie in Beispiel 1 zerkleinert. Dann wurde von einer auf Hallimasch gewonnenen Kultur von Trauermücken und Pilzmücken eine Menge von etwa 20 g zu dem Röhrlingssubstrat zugesetzt. Es zeigte sich eine deutlich schnellere Entwicklung der Maden als dies in Beispiel 1 der Fall war. Die Gewinnung des Extraktes erfolgte wie im Beispiel 1.

Das erhaltene Produkt zeigte etwa die gleiche Wirkungskombination A + B wie das Produkt von Beispiel 1.

**Beispiel 4**

1 kg zerkleinertes Fruchtkörpermaterial des Maronenröhrlings wurde wie in Beispiel 1 hergestellt und 48 Stunden bei etwa 20°C offen stehengelassen. Das Produkt färbte sich dunkelbraun bis tiefschwarz. Dann wurden 750 g reines Olivenöl zu dem Material zugesetzt und das erhaltene Gemisch sofort auf 100°C erhitzt, worauf es etwa 12 Stunden bei 70 bis 80°C gehalten wurde. Das Produkt wurde dann eine Woche in einem geschlossenen Behälter stehengelassen. Anschließend wurde die Ölschicht auf einer Zentrifuge abzentrifugiert.

Das erhaltene Hautöl wird zur Regenerierung und Straffung gealterter Haut und zur Beseitigung von Falten angewandt. Es wird hierzu leicht in die Haut eingerieben. In zahlreichen Fällen wird neben einer Straffung der Haut auch die Beseitigung von Hautflecken bewirkt.

### Beispiel 5

200 g Junge madenfreie Maronenröhrlinge wurden wie in Beispiel 1 verarbeitet und der Extrakt gewonnen.

Der 40 %-ige Extrakt zeigte Wirksamkeit gegen Haarausfall, Schuppenbildung und Fettfluß, was als Wirkung A bezeichnet wird.

### Vergleichsbeispiel A

Etwa 10 g isolierte Maden der Pilzmücke und der Trauermücke wurden mit 10 g 70 %-igem Äthanol wie in Beispiel 1 extrahiert und der Extrakt filtriert.

Bei dem im Vergleichsbeispiel erhaltenen Extrakt ließen sich nicht die vorstehend angegebenen Wirkungen A oder B feststellen.

### Beispiel 6

0,5 kg frisches grob zerkleinertes Moos aus etwa gleichen Teilen Dicranum und Bryum wurden mit 2,5 kg 45 %-igem Äthanol während 1 Stunde digeriert und sofort abgepreßt. Der Extrakt wurde auf eine Alkoholkonzentration von 40 Volumen-% eingestellt und zeigte die vorstehende Wirkung A sowie Anzeichen einer Wirksamkeit B.

### Beispiel 7

40 Volumenteile des Extraktes von Beispiel 1, 40 Volumenteile des Extraktes von Beispiel 6 und 20 Volumenteile des Extraktes aus 0,5 kg Beinwell mit 50 %-igem Äthanol wurden vereinigt und auf eine Alkoholkonzentration von 40 Volumen-% eingestellt.

Das Mittel zeigte eine ausgezeichnete Kombination der Wirksamkeiten A und B.

### Beispiel 8

Zu dem Mittel nach Beispiel 7 wurden 2 Gew.-% Allantoin (als Feststoff) zugesetzt.

Die Wirkungskombination A + B konnte dadurch weiterhin stabilisiert werden.

### Beispiel 9

Es wurde entsprechend Beispiel 2 gearbeitet, jedoch als Extraktionsmittel eine Kombination aus Wasser, Äthanol und Isopropanol mit einem Volumenverhältnis von 20 : 40 : 40 angewandt und der erhaltene Extrakt auf eine Alkoholvolumenkonzentration (Äthanol : Isopropanol = 1 : 1) von 40 Volumen-% eingestellt.

Das Mittel zeigte die Wirksamkeitskombination A + B.

### Vergleichsbeispiel B

Ein 40 %-iger Äthanolextrakt aus Beinwell und Äthanol zeigte keinerlei Wirkung bei Haarproblemen, und zwar weder im Hinblick auf die Wirksamkeit A noch auf die Wirksamkeit B, obwohl sich dieser Extrakt beim Zusatz gemäß den erhaltenen Mitteln nach Beispiel 2 und 7 als durchaus wirkungsvoll erwies, falls entsprechend den dort angegebenen Arbeitsweisen gearbeitet wurde.

### Patentansprüche

1. Biologisches Mittel zur Behandlung der Kopfhaut und der Haare, gekennzeichnet durch einen Extrakt auf der Basis hautverträglicher Alkohole oder Öle von

1) Fruchtkörpern von ungiftigen Ständerpilzen (Basidiomyceten), ausgenommen Calvatia maxima, und
2) Insektenlarven, die sich von Pilzen ernähren, sowie deren Stoffwechselprodukten und gegebenenfalls
3) weiteren Zusätzen.

2. Biologisches Mittel nach Anspruch 1, gekennzeichnet durch den Gehalt eines oder mehrerer weiterer Zusätze aus der Gruppe von alkoholischen Extrakten aus Moosen, alkoholischen Extrakten aus Beinwell (Symphytum officinale) und Allantoin.

3. Biologisches Mittel nach Anspruch 1, dadurch gekennzeichnet, daß der Alkohol aus Äthanol oder Isopropanol besteht.

4. Biologisches Mittel nach Anspruch 1 bis 3, dadurch gekennzeichnet, daß die Pilzfruchtkörper aus solchen der Familien Agaricales und/oder Boletales bestehen.

5. Biologisches Mittel nach Anspruch 4, dadurch gekennzeichnet, daß die Pilzfruchtkörper aus solchen von Maronenröhrling (Xerocomus badius) und/oder Hallimasch (Armillariella mellea) bestehen.

6. Biologisches Mittel nach Anspruch 1 bis 5, dadurch gekennzeichnet, daß die Insektenlarven aus Fliegenlarven bestehen.

7. Biologisches Mittel nach Anspruch 6, dadurch gekennzeichnet, daß die Fliegenlarven aus solchen der Familien Lycoriidae (Sciaridae, Trauermücken) und/oder Fungivoridae (Mycetophilidae, Pilzmücken) bestehen.

8. Biologisches Mittel nach Anspruch 1 bis 7, dadurch gekennzeichnet, daß der alkoholische Extrakt aus Moosen aus solchen von Schönem Widertonmoos (Polytrichum formosum), Gabelzahnmoos (Dicronum scoparium), Silber-Birnmoos (Bryum argenteum) oder Schwarzem Eichhornmoos (Lencodon scinroides) besteht.

9. Verfahren zur Herstellung des biologischen Mittels zur Behandlung der Haare und der Haut nach Anspruch 1 bis 8, dadurch gekennzeichnet, daß ein Gemisch aus zerkleinerten Fruchtkörpern von ungiftigen Ständerpilzen (Basidiomyceten), ausgenommen Calvatia maxima, und Insektenlarven, die sich von Pilzen ernähren, während eines Zeitraums von einigen Stunden bis zu einigen Tagen stehengelassen wird und anschließend mit einem hautverträglichen Alkohol oder Öl kontaktiert wird und der erhaltene Extrakt abgetrennt wird und gegebenenfalls mit den weiteren Zusätzen aus der Gruppe von alkoholischen Extrakten aus Moosen, alkoholischen Extrakten aus Beinwell (Symphytum officinale) und/oder Allantoin versetzt wird.

## Claims

1. Biological composition for scalp and hair treatment, characterized by an extract on the basis of skin-compatible alcohols or oils from

1) receptacles of non-toxic basidia mushrooms (basidiomycetes) with the exception of calvatia maxima, and
2) larvas of insects feeding on mushrooms as well as products of metabolism thereof and if desired
3) further additives.

2. Biological composition according to claim 1, characterized by the content of one or more further additives from the group of alcoholic extracts from mosses, alcoholic extracts from comfrey (symphytum officinale) and allantoin.

3. Biological composition according to claim 1, characterized in that the alcohol consists of ethanol or isopropanol.

4. Biological composition according to claims 1 to 3, characterized in that the mushroom receptacles consist of such of the families agaricales and/or boletales.

5. Biological composition according to claim 4, characterized in that the mushroom receptacles consist of such of cepe (xerocomus badius) and/or honey mushroom (armillariella mellea).

6. Biological composition according to claims 1 to 5, characterized in that the larvae of insects consist of larvae of flies.

7. Biological composition according to claim 6, characterized in that the larvae of flies consist of such of the families lycoriidae (sciaridae, fickle midges) and /or fungivoridae (mycetophilidae, agaric midges).

8. Biological composition according to claims 1 to 7, characterized in that the alcoholic extract from mosses consists of such of golden maiden hair (polytrichum formosum), crown-beard moss (dicronum scoparium), thread moss (bryum argenteum) or black squirrel moss (lencodon scinroides).

9. Process for preparing the biological composition for hair and skin treatment according to claims 1 to 8, characterized in that a mixture of comminuted receptacles of non-toxic basidia mushrooms (basidiomycetes) with the exception of calvatia maxima, and larvae of insects feeding on mushrooms is left standing during a time of several hours up to several days and subsequently is contacted with a skin-compatible alcohol or oil and the resulting extract is separated and if desired the further additives from the group of alcoholic extracts from mosses, alcoholic extracts from comfrey (symphytum officinale) and/or allantoin are added thereto.

## Revendications

1. Produit biologique pour le traitement du cuir chevelu et des cheveux, caractérisé par un extrait à base d'alcools ou d'huiles compatibles avec la peau, de

1) fructifications de basidiomycètes non toxiques, à l'exception de Calvatia maxima, et
2) larves d'insectes qui se nourissent de champignons, ainsi que de leurs métabolites, et éventuellement,
3) d'autres additifs.

2. Produit biologique selon la revendication 1, caractérisé par la teneur en un ou plusieurs autres additifs appartenant à la catégorie d'extraits alcooliques de mousses, d'extraits alcooliques de consoude (Symphytum officinale) et d'allantoïne.

3. Produit biologique selon la revendication 1, caractérisé en ce que l'alcool consiste en l'éthanol ou l'isopropanol.

4. Produit biologique selon l'une quelconque des revendications 1 à 3, caractérisé en ce que les fructifications consistent en fructifications de champignons appartenant à l'ordre des agaricales et/ou à la famille des bolets.

5. Produit biologique selon la revendication 4, caractérisé en ce que les fructifications de champignon consistent en celles du bolet fauve (Xerocomus badius) et/ou de l'armillaire de miel (Armillariella mellea).

6. Produit biologique, selon l'une quelconque des revendications 1 à 5, caractérisé en ce que les larves d'insectes consistent en larves de mouches.

7. Produit biologique selon la revendication 6, caractérisé en ce que les larves de mouches consistent en celles appartenant aux familles des Sycoriidae (Sciaridae, Tipule funèbre) et ou des fungivores (mycétophilides, mouche des champignons).

8. Produit biologique selon l'une quelconque des revendications 1 à 7, caractérisé en ce que l'extrait alcoolique de mousses consiste en extraits de polytrie (Polytrichum formosum), dicranum (Dicronum scoparium)) "Silberbirnmoos (Byrum argenteum) ou eichhornia noir (Lendocon scinroides).

9. Procédé pour la préparation du produit biologique pour le traitement des cheveux et de la peau selon l'une quelconque des revendications 1 à 8, caractérisé en ce qu'on laisse reposer pendant une période allant de quelques heures à quelques jours un mélange de fructifications fragmentées de basidiomycètes non toxiques (à l'exception de Calvatia maxima) et de larves d'insectes qui se nourissent de champignons, et on le met ensuite en contact avec un alcool ou une huile compatible avec la peau, puis on sépare l'extrait obtenu et on y a joute éventuellement les autres additifs appartenant à la catégorie d'extraits alcoliques de mousses, d'extraits alcooliques de consoude (Symphytum officinale) et/ou d'allantoïne.